# EUROPÄISCHE PATENTSCHRIFT

(11) **EP 1 411 977 B1**
(45) Veröffentlichungstag und Bekanntmachung des Hinweises auf die Patenterteilung: **18.04.2007**
(21) Anmeldenummer: 00991649.5
(22) Anmeldetag: 29.12.2000
(51) Int. Cl.: A61K 39/02, A61K 39/116, A61K 39/295, A61K 39/102, A61P 37/00, A61P 37/08, A61P 11/06, A61P 31/04

(54) **ZUSAMMENSETZUNG, ENTHALTEND BAKTERIELLE ANTIGENE, ZUM VORBEUGEN UND BEHANDELN VON ALLERGISCHEN ERKRANKUNGEN**
COMPOSITION CONTAINING BACTERIAL ANTIGENS, USED FOR THE PROPHYLAXIS AND THE TREATMENT OF ALLERGIC DISEASES
COMPOSITION DE PREVENTION ET DE TRAITEMENT DES TROUBLES ALLERGIQUES

(30) Priorität: 30.12.1999 DE 19963840
(43) Veröffentlichungstag der Anmeldung: 28.04.2004
(73) Patentinhaber: Bufe, Albrecht, Prof. Dr. med., 20255 Hamburg (DE); Holst, Otto, Prof. Dr. rer. nat., 23843 Bad Oldesloe (DE)
(72) Erfinder: Nowak, Dennis, Dr., 82152 Krailling (DE); Riedler, Josef, Dr., 5500 Bischofshofen (AT); Braun-Fahrländer, Charlotte, Prof. Dr., 4052 Basel (CH); von Mutius, Erika, Dr., 82319 Leutstetten (DE)
(74) Vertreter: Engels, Barbara
(86) Internationale Anmeldenummer: PCT/EP2000/013330
(87) Internationale Veröffentlichungsnummer: WO 2001/049319

(56) Entgegenhaltungen:
- WO-A-91/12818
- WO-A-96/00579
- WO-A-99/66947
- GIRARD J P ET AL: "[ Asthma and bacterial infections]. Asthme et infections bacteriennes." SCHWEIZERISCHE MEDIZINISCHE WOCHENSCHRIFT. JOURNAL SUISSE DE MEDECINE, (1984 JUN 23) 114 (25) 890-2. , XP000996040
- OEHLING A: "Bacterial immunotherapy in bronchial asthma." JOURNAL OF INVESTIGATIONAL ALLERGOLOGY AND CLINICAL IMMUNOLOGY, (1997 JAN-FEB) 7 (1) 14-9. REF: 33 , XP000996031
- CHAPMAN J A: "Update on airborne mold and mold allergy." ALLERGY AND ASTHMA PROCEEDINGS, (1999 SEP-OCT) 20 (5) 289-92. REF: 17 , XP000995642
- POULTRY SCIENCE, Bd. 60, Nr. 3, 1981, AIRBORNE MICRO FLORA OF POULTRY HOUSES
- AMERICAN REVIEW OF RESPIRATORY DISEASE, Bd. 40, Nr. 2, 1989, AEROALLERGENS IN DAIRY BARNS NEAR COOPERSTOWN NEW YORK AND ROCHESTER MINNESOTA USA
- SCANDINAVIAN JOURNAL OF WORK ENVIRONMENT AND HEALTH, Bd. 21, Nr. 3, 1995, PREVALENCE OF MICROFUNGI IN FINNISH COW BARNS AND SOME ASPECTS OF THE OCCURRENCE OF WALLEMIA SEBI AND FUSARIA
- NOLAN ET AL: "A combined liquid Hib (PRP-OMP), hepatitis B, diphteria, tetanus and whole-cell pertussis vaccin: uncontrolled preliminary clinical trial of immunogenicity and reactogenicity." VACCINE, Bd. 16, Nr. 20, Seiten 2085-2089,

## Beschreibung

Die vorliegende Erfindung betrifft die Verwendung einer Zusammensetzung, die mindestens ein aerogen übertragbares mikrobielles Antigen umfasst, das typischerweise in Stallluft auftritt, zum Vorbeugen und Behandeln von allergischen Reaktionen und ein Verfahren zum Herstellen dieser Zusammensetzung.

Als Allergien bezeichnet man unangemessene Reaktionen des Körpers auf nicht schädliche fremde Substanzen. Diese Reaktionen laufen genauso wie die normale immunantwort gegen einen Krankheitserreger ab. Da im Falle einer Allergie das Allergen selbst unschädlich ist, beruhen die Krankheitserscheinungen ausschließlich auf der immunreaktion. Es werden mehrere Typen der allergischen Reaktion unterschieden. Allergische Reaktionen vom Typ I, zu denen Asthma bronchiale, atopische Dermatitis und Heuschnupfen gehören, sind am weitesten verbreitet Obgleich diese Krankheiten unterschiedliche Symptome aufweisen, liegen ihnen ähnliche immunologische Mechanismen zugrunde.

Verantwortlich für die Symptome der Allergien vom Typ I sind Antikörper vom Typ IgE. Die Ursachen und Risiken für die Ausbildung einer allergischen Erkrankung sind noch nicht vollständig aufgeklärt. Es ist jedoch sicher, dass ein wesentlicher Schritt in der Entstehung einer solchen Erkrankung in der Prägung des Immunsystems, insbesondere in der Reifung der Th1- und Th2-Zellen, liegt. Für die Bildung der IgE-Antkörper sind Interleukin 4 (IL-4) produzierende Th2-Zellen notwendig, während Interferon-γ (INF-γ) produzierende Th1-Zellen auf die Bildung von lgE-Antikörpern hemmend wirken. Beide Zelltypen sind in einem ausgewogenen Verhältnis für die lmmunabwehr notwendig. Th1-Zellen sind für die lmmunantwort gegen die verschiedensten Infektionskrankheiten verantwortlich, während Th2-Zellen bei der Abwehr bestimmter Nematoden-Infektionen benötigt werden. Im Fall einer allergischen Erkrankung ist jedoch dieses ausgewogene Th1/Th2-Zellverhältnis in Richtung einer Th2-dominierten Immunantwort verschoben.

Die Ausprägung des Immunsystems wird neben der individuellen genetischen. Prädisposition von der externen Stimulation bestimmt. Die Reifung der Th1 -Zellen wird durch die Präsenz von Antigenen (z.B. Endotoxinen) initiiert, indem diese über Antigenpräsentierende Zellen wie Monozyten und dendritische Zellen eine Ausschüttung von IL-12 bewirken, welches eine Differenzierung von naiven T-Zellen zu Th1-Zellen zur Folge hat.

Man nimmt an, dass vor allem die Prägung des Immunsystems in den frühen Lebensjahren von entscheidender Bedeutung für die Allergieanfälligkeit ist Tierexperimentelle Untersuchungen haben ergeben, dass die Immunantwort Neugeborener hauptsächlich auf Th2-Zellen beruht, d.h. dass die Ausprägung der gesunden Th1-dominierten immunantwort der externen Prägung durch Antigene bedarf. Somit kann umgekehrt angenommen werden, dass durch die unzureichende Prägung der naiven Th-Zellen in Th1-Zellen eine wichtige Voraussetzung für allergische Erkrankungen vom Typ I geschaffen wird. Tierexperimente belegen, dass wesentliche Initiatoren des Reifungsproozesses der Th1-Zellen mikrobieller Natur sind und dass bar Ausschluss solcher Stimuli durch eine keimfreie Umgebung das Immunsystem im Th2-Zell dominierten Zustand belassen wird.

Verschiedene Untersuchungen haben ergeben, dass das unmittelbare Lebensumfeld während der frühen Kindheit einen wesentlichen Einfluss auf die spätere Anfälligkeit für allergische Erkrankungen hat Es ist bekannt, dass ein industrialisierter westlicher Lebensstil mit dem Anstieg von allergischen Erkrankungen korreliert. Umgekehrt scheinen schlechtere hygienische Bedingungen dem Auftreten von Allergien vorzubeugen. Verschiedene epidemiologische Untersuchungen haben gezeigt, dass allergische Erkrankungen seltener in ländlichen als in städtischen Bevölkerungsgruppen vorkommen. Es konnte gezeigt werden, dass Kinder, die unmittelbar auf einem Bauemhof aufwuchsen, verglichen mit Kindern aus demselben Dorf, die nicht auf einem Bauemhof lebten, weit weniger allergieanfällig waren. Andere Faktoren, wie die Anzahl der Geschwister, die Heizungstechnik oder das Vorhandensein von Haustieren, wiesen eine geringere Korrelation mit dem Auftreten allergischer Erkrankungen auf als die bäuerliche Umgebung. Andere Studien haben gezeigt, dass der Schutz vor allergischen Erkrankungen, der durch das Aufwachsen In einem ländlichen Umfeld entsteht, bis in das Erwachsenenalter bestehen bleibt Die Ursachen für diesen Schutzeffekt konnten bis jetzt nicht befriedigend aufgeklärt werden. Allgemeine Faktoren wie eine erhöhte Pollenbelastung oder eine vergleichsweise geringe Schadstoffbelastung der Luft sind jedoch als Ursache nicht sehr wahrscheinlich.

Die Möglichkeiten zur Therapie und Prävention allergischer Erkrankungen sind begrenzt Zumeist werden bei bereits manifestierten allergischen Erkrankungen (wie z.B. Heuschnupfen) medikamentöse Therapien und Desensibilisierungtherapien angewandt. Die Erfolge dieser Therapien sind überaus unterschiedlich. Vor allem bei chronischen Erkrankungen der Atemwege bestehen kaum Chancen auf Heilung, daher müssen sich die Patienten einer Dauertherapie unterziehen. Weiterhin sind die herkömmlichen Behandlungen oft mit beträchtlichen Nebenwirkungen behaftet.

Durch den starken Anstieg der Allergieerkrankungen in den letzten Jahren und die begrenzte Anzahl wirksamer Therapien besteht ein großer Bedarf nach neuen wirlwngsvollen Präventions- und Behandlungsstrategien. Tierversuche haben gezeigt, das die intranasale Infektion mit Mycobacterium- Partikeln Allergie-Induzierte Eosinophillie vermindert, allerdings einhergehend mit einer massiven infektiösen Erkrankung der Tiere. Ein anderer Ansatz ist die präventive Desensibüisierung gegen bestimmte Altergene. Es gibt jedoch bisher keine Möglichkeit, das Risiko für Allergien Insgesamt zu senken.

Aufgabe der vorliegenden Erfindung ist es, ein Mittel zum Vorbeugen und zum Behandeln von allergischen Beschwerden zur Verfügung zu stellen.

Die Aufgabe wird erfindungsgemäß durch Verwendung eines Zusammensetzung gemäß Anspruch 1 gelöst, Weiterhin umfasst die Erfindung ein Verfahren zum Herstellen dieser Zusammensetzung.

Die erfindungsgemäße Verwendung des Zusammensetzung ist geeignet, das kindliche Immunsystem so zu stimulieren, dass das Risiko zur Ausbildung einer allergischen Erkrankung vermindert werden kann. Die Erfindung beruht auf der Erkenntnis, dass der oben beschriebene immunologische Schutzeffekt einer bäuerlichen Umgebung gegen allergische Erkrankungen auf dem intensiven Kontakt der Kinder mit Stalltieren beruht. Es ist weiterhin erkannt worden, dass die erhöhte mikrobielle Belastung der bäuerlichen Umgebung für eine Stimulierung des kindlichen lmmunsystems verantwortlich ist, so dass ein für die Verminderung von allergischen Erkrankungen günstiges Verhältnis von Th2- zu Th1-Zellen induziert wird.

Tierställe weisen gegenüber dem häuslichen Bereich eine stark erhöhte Konzentration bakterieller Antigene, insbesondere Endotoxine, auf. Erhöhte Endotoxinwerte werden auch in den Matratzen der Kinder gefunden, die auf einem Bauernhof mit Tierhaltung aufwachsen, sowie der Kinder, die einen regelmäßigen Kontakt zu Stalltieren haben, aber nicht auf einem Hof leben, wenn deren Matratzen mit den Matratzen der Kinder aus der Kontrollgruppe des gleichen Dorfes, die keinen Kontakt zu Stalltieren haben, verglichen werden. Das bedeutet, dass die hohe Endotoxinbelastung, die mit einer erhöhten mikrobiellen Antigen-Belastung gleichzusetzen ist, sich bei diesen beiden Gruppen im häuslichen Bereich fortsetzt und somit eine fortwährende Stimulierung des Immunsystems gewährleistet. Kinder, die einen regelmäßigen Kontakt zu Stalltieren haben, zeigen eine signifikant geringere Anfälligkeit für allergische Erkrankungen als die Kontrollgruppe, die keinerlei Kontakt zu den Stalltieren hat

Die vorliegende Erfindung ermöglicht eine Simulation dieses intensiven Stalltierkontaktes und damit eine günstige Prägung des kindlichen Immunsystems, mit der eine verminderte Anfälligkeit für allergische Erkrankungen erreicht wird.

Die erfindungsgemäß zu verwendende Zusammensetzung enthält ein oder mehrere bakterielle Antigene. Die Zusammensetzung ist weiterhin dadurch gekennzeichnet, dass sie zusätzliche Antigene enthält die aus der Gruppe der Pilze, Viren und Protozoen oder Fragmente derselben ausgewählt sind.

Die Bakterien können allein oder in Kombination aus folgender Gruppe ausgewählt werden: *Bordetella* spp, bevorzugt *Bordetella broncheseptic; Brucella* spp, bevorzugt *Brucella suis, Corynebacterium* spp, bevorzugt *Corynebacterium equi, Erysipelothrix* spp, bevorzugt *Erysipelothrix rhusiopathiae, Escherichia* spp, bevorzugt *Escherichia coli, Haemophilus* spp, bevorzugt *Haemophilus gallinarus, Haemophilus parasuis, Haemophilus pleuropneumoniae, Listeria* spp, bevorzugt *Listeria monocytogenes, Leptospira* spp, bevorzugt *Leptospira pomona, Mycobacterium* spp, bevorzugt *Mycobacterium avium, Mycobacterium bovis, Mycobacterium tuberculosis, Mycoplasma* spp, bevorzugt *Mycoplasma gallispeticum, Mycoplasma hyorhinus, Mycoplasma suipneumoniae, Pasteurella* spp, bevorzugt *Pasteurella multocida, Pasteurella pseudotuberculosis, Salmonella* spp, bevorzugt *Salmonella pullorum, Salmonella typhimurium, Staphylococcus* spp, bevorzugt *Staphylococcus aureus, Streptococcus* spp, bevorzugt *Streptococcus suis type ll* und *Coxiella* spp, bevorzugt *Coxiella burnetii.*

Die Zusammensetzung kann insbesondere die folgenden Kombinationen bakterieller Antigene enthalten: *Mycobacterium spp* und *Staphylococcus spp, Escherichia spp* und *Salmonella spp, Mycobacterium spp* und *Salmonella spp, Mycobacterium spp* und *Escherichia spp, Staphylococcus spp* und *Escherichia spp, Staphylococcus spp* und *Salmonella spp, Listeria spp und Mycobacterium spp , Listeria spp und Staphylococcus spp, Usteria spp und Escherichia spp, Listeria spp und Salmonella spp.* Die bakte nellen Antigene können entweder als Fragmente der Bakterien, isolierte Proteine oder Peptide enthalten sein.

Die Zusammensetzung kann außerdem nur aus atypischen Mycobakterien oder Fragmenten von atypischen Mycobakterien zusammengesetzt sein, bevorzugt aus *Mycobacterium bovis* und *Mycobacterium avium.*

Die Zusammensetzung kann außerdem nur aus *Listeriae spp* oder Fragmenten von *Listeriae spp* zusammengesetzt sein, bevorzugt aus *Listeria monocytogenes.*

Die Zusammensetzung kann weiterhin Pilze und oder deren Sporen allein oder in Kombination umfassen, wobei die Pilze aus der *Aspergillus flavus, Aspergillus fumigatus, Aspergillus nidulans, Aspergillus niger, Coccidioides immitis, Cryptococcus neoformans, Histoplasma farcinorum* umfassenden Gruppe ausgewählt sind.

Die verwendeten Mikroorganismen zur Herstellung der erfindungsgemäßen Zusammensetzung können in lebendem oder abgetöteten Zustand vorliegen. Die Mikroorganismen können in ihrer Gesamtheit oder selektiv durch bekannte Verfahren abgetötet werden, beispielsweise durch Zusetzen von fungiziden oder fungistatischen, viriziden oder virustatischen, bakteriziden oder bakteriostatischen Stoffen, durch ultraviolette oder radioaktive Strahlung, durch Hitze, Lyophylisierung oder Kältesterilisation.

Die Zusammensetzung besteht erfindungsgemäß hauptsächlich aus isolierten Fragmenten der Mikroorganismen. Die isolierten Fragmente der Mikroorganismen können Zelltrümmer, Membranstücke, Organellen, einzelne Proteine oder Peptide dieser Proteine oder Membranlipide umfassen. Die Fragmente können durch allgemein bekannte Aufschlußverfahren und Aufreinigungsverfahren erhalten werden, so z.B. durch chemische Lyseverfahren, Ultraschall oder Druck. Die Fragmente können weiterhin ganz oder teilweise durch bekannte biochemische Verfahren aufgereinigt werden, d.h. Nukleinsäuren und andere nicht erwünschte Bestandteile können ganz oder teilweise z. B. durch Zentrifugation, chromatographische Verfahren, Gelfiltration und/oder Aussalzung abgetrennt werden.

In einer bevorzugten Ausführung umfasst die erfindungsgemäße Zusammensetzung insbesondere atypische Mycobakterien, Fragmente davon oder isolierte Proteine oder. Peptide, die mycobakteriellen Antigenen entsprechen, die wie oben beschrieben hergestellt sein können.

In einer bevorzugten Ausführung umfasst die erfindungsgemäße Zusammensetzung Insbesondere *Listeria spp,* Fragmente davon oder isolierte Proteine oder Peptide, die Antigenen dieser Familie entsprechen, die wie oben beschrieben hergestellt sein können.

Die in der erfindungsgemäßen Zusammensetzung enthaltenen Antigene können beispielsweise Proteine und/oder Peptide sein, die durch rekombinante DNA-Techniken erzeugt wurden. Die Proteine oder deren Peptide können auch synthetisch hergestellt worden sein. Die Zusammensetzung kann weiterhin Mutanten und Varianten der ursprünglichen Antigene enthalten.

Bevorzugte Antigene sind z.B. isolierte Proteine oder Protein-hattige Komplexe, die aus der Gruppe der Endotoxine, viralen Hüllproteine, Zellwandproteine und cytosolische Proteine und Glykoproteinen ausgewählt sind. Ein bevorzugtes Endotoxin ist das Lipopoitsacchand (LPS), welches in der äußeren Membran von Gram-negativen Bakterien vorkommt Ein weiteres bevorzugtes Endotoxin ist das Staphylococcus Endotoxin B (SEB). Weiterhin bevorzugt ist die Lipoteichonsäure (LTA) Gram-positiver Bakterien. Weiterhin bevorzugt ist das Upoarabinomannan (LAM) aus Mycobacteria.

Die zur Herstellung der Zusammensetzung verwendeten Mikroorganismen oder die Fragmente derselben können kleiner oder gleich 100 µm, bevorzugt jedoch kleiner als 50 µm sein, noch mehr bevor zugt kleiner als 22 µm sein. Je nach Applikationsfonn können die Mikroorganismen oder deren Fragmente auch kleiner als 5 µm sein.

Die Zusammensetzung kann als wässrige Lösung vorliegen. Die Lösung kann vorzugsweise isotonisch sein, d.h. eine physiologische Salzkonzentration enthalten. Die Lösung kann weiterhin unbedenkliche bzw. annehmbare natürliche oder synthetische Zusätze oder Hilfstoffe enthalten, wie Konservierungsstoffe, Stabilisatoren. Geruchs- und Geschrnacicskomgentien, pharmazeutisch unbedenkliche Trägerstoffe. Emulgatoren, Verdünnungsmittel. Weiterhin kann die Lösung fungizide oder fungistatische, bakterizid oder bacteriostatische, virizide oder virustatische Wirkstoffe enthalten.

Die Zusammensetzung kann als trockenes Konzentrat vorliegen, d.h. sie kann von flüchtigen und/oder flüssigen Bestandteilen, insbesondere Wasser, befreit sein.

Die erfindungsgemäß zu verwendende Zusammensetzung kann als Lyophylisat vorliegen.

Die erfindungsgemäß zu verwendende Zusammensetzung kann weiterhin als Aerosol vorliegen. Das Aerosol besteht aus Meinen festen oder flüssigen Teilchen, die durch einen Inhalator, einen Vernebler oder ein Beatmungsgerät erzeugt werden. Die Teilchen des Aerosols können aus der Zusammensetzung allein oder der Zusammensetzung in Verbindung mit einem geeigneten Trägermaterial bestehen. Dabei kann das Aerosol eine Teilchengröße von bis zu 100 µm für die instillative Anwendung In der Nase haben. Weiterhin kann das Aerosol Teilchen einer Größe von bis zu 10 µm, bevorzugt unter 5 µm für die inhalative Anwendung enthalten.

Die vorliegende Erfindung betrifft die Verwendung der Zusammensetzung in ihren verschiedenen vorherstehend beschriebenen Ausführungen zur Vorbeugung oder Behandlung von allergischen Beschwerden. Insbesondere kann die Zusammensetzung zur Vorbeugung oder Behandlung von Heuschnupfen, atopischer Dermatitis, Asthma bronchiale oder Nahrungsmittelallergien verwendet werden.

Die vorliegende Erfindung ist insbesondere geeignet, das naive Immunsystem zu prägen. Daher kann die Zusammensetzung erfindungsgemäß Säuglingen und Kindern in den ersten Lebensjahren wiederholt verabreicht werden, die aufgrund einer positiven Familienanamnese ein deutlich erhöhtes Risiko aufweisen, eine allergische Erkrankung zu entwickeln. Unter einer positiven Familienanamnese kann verstanden werden, wenn ein Elternteil oder beide Elternteile und/oder ein oder mehrere Geschwister, und/oder Verwandte zweiten Grades bereits an einer allergischen Erkrankung leiden oder allergische Symptome gezeigt haben, insbesondere Heuschnupfen, Asthma, atopische Dermatitis oder Nahrungsmittelallergien.

Weiterhin ist die Zusammensetzung geeignet, an Säuglinge und Kinder verabreicht zu werden, die bereits erste Anzeichen einer Allergie aufweisen, insbesondere erste Symptome von Heuschnupfen. Asthma, atopischer Dermatitis, positiver IgE-Reaktion auf Nahrungsmittel, Nahrungsmtttelaltergien, insbesondere Nahrungsmittelallergien auf Kuhmilch und Hühnereiweiß. Weiterhin können Kinder und Säuglinge, die ein erhöhtes Risiko haben, an einer Allergie zu erkranken, auf Grund genetischer Marker durch RFLP- und PCR-Techniken identifiziert werden. Weiterhin können diese Kinder durch eine verringerte Produktion von INF-γ nach Stimulierung der Leukozyten identifiziert werden.

Die Zusammensetzung ist geeignet, zur Vorbeugung und Behandlung von allergischen Erkrankungen an Kinder und Erwachsene verabreicht zu werden.

Insbesondere ist die Zusammensetzung geeignet, zur Vorbeugung von allergischen Erkrankungen an Kinder und Säuglinge im Alter von bis zu fünf Jahren, bevorzugt bis zu zwei Jahren verabreicht zu werden.

Die erfindungsgemäße Zusammensetzung kann intranasal, instillativ oder inhalativ verabreicht werden. Hierbei kann die Zusammensetzung mit einem handelsüblichen inhalator, Vernebler oder Beatmungsgerät verabreicht werden. Das Aerosol kann kalt oder bis auf Körpertemperatur angewärmt verabreicht werden. Das Aerosol kann so appliziert werden, dass es einen Antigengehalt von 0.5 - 20.000 Antigen-Partikel pro Liter Luft, bevorzugt 3 - 10.000 Antigen-Partikel pro Liter Luft enthält; als Antigen-Partikel gelten hierbei entweder intakte Mikroorganismen oder Fragmente, gegebenenfalls in Verbindung mit Tragerpartikeln, z.B. Staub oder Strohteilchen.

Die Zusammensetzung kann in regelmäßigen Abständen über einen längeren Zeitraum verabreicht worden, um so eine kontinuierliche Stimulation des Immunsystems zu erreichen. Die Zusammensetzung kann beispielsweise über bis zu 10 Jahre hinweg, 1-21 mal wöchentlich, vorzugsweise 7-14 mal wöchentlich verabreicht werden. Die Dauer der Behandlung kann zwischen 5 bis 120 min, vorzugsweise zwischen 15 bis 60 min liegen.

Die vorliegende Erfindung umfasst außerdem ein Verfahren zum Herstellen der vorstehend beschriebenen Zusammensetzung. Zum Herstellen der Zusammensetzung kann ein Luftfilter verwendet werden, durch den die Stallluft gefiltert wird. Dabei kann der Filter aus einem Material mit großer Oberfläche bestehen, z.B. einem Membranfilter oder einem Granulat, an dem die Antigenpartikel reversibel adsorbiert werden. Zum Herstellen der erfindungsgemäßen Zusammensetzung kann auch ein Impinger verwendet werden; hierbei können die aerogenen Antigen-Partikel direkt in eine wässrige Lösung, vorzugsweise in eine isotonische Lösung gesammelt werden. Zum Herstellen der Zusammensetzung kann erfindungsgemäß auch ein impaktor, bevorzugt ein Kaskaden-impaktor verwendet werden, mit dem die Antigen-Partikel der Stailluft entnommen werden. Hierbei werden die Antigen-Partikel durch selektive Abscheidung gesammelt. Die Sammlung kann mehrstufig, vorzugsweise 6 bis 9 stufig, auf Platten, die mit Agar beschichtet sein können, in Abhängigkeit von der Größe der Partikel erfolgen, wobei die Größe zwischen 20 nm bis 22 µm, vorzugsweise zwischen 60 nm bis 16 µm liegt Für die erfindungsgemäß zu verwendende Zusammensetzung können ein bis sämtliche Fraktionen ausgewählt werden, bevorzugt die Fraktionen (Platten) mit Antigen-Partikeln kleiner als 10 µm, noch mehr bevorzugt kleiner als 5 µm für die inhalative Applikation oder bevorzugt die Fraktionen (Platten) mit Antigen-Partikeln kleiner als 22 µm für die installative Anwendung. Anschließend werden die Antigen-Partikel von den Platten abgekratzt, in einer wässrigen Suspension aufgenommen und aufgeschlossen. Die Suspension wird einer Ultraschallbehandiung unterzogen, so dass die Koagulation der enthaltenen Partikel verhindert wird.

Die wässrige Suspension, umfassend die erfindungsgemäße Zusammensetzung, kann direkt mit einem Vernebler, Inhalator oder Beatmungsgerät appliziert werden.

Das Verfahren zum Herstellen der erfindungsgemäße zu verwendenden Zusammensetzung kann Maßnahmen umfassen, die Zusammensetzung von den flüchtigen und/oder flüssigen Bestandteilen, insbesondere Wasser, abzutrennen und ein Trockenkonzentrat zu erhalten. Die Abtrennung kann beispielsweise durch Trocknen mit Luft, insbesondere mit vorgetrockneter Luft, Trocknen durch überkritisches CO₂, Sprühtrocknen oder Einengen im Vakuum erfolgen.

Das Verfahren zum Herstellen der erfindungsgemäßen Zusammensetzung kann weiterhin Sterilisationsschritte umfassen. Die Zusammensetzung kann wahlweise mit fungiziden oder fungistatischen, bakteriziden oder bacteriostatischen, viriziden oder virustatischen Mitteln behandelt werden, beispielsweise durch Zusetzen von fungiziden oder fungistatischen, bakteriziden oder bacteriostatischen, viriziden oder virustatischen Stoffen, durch ultraviolette oder radioaktive Strahlung oder durch Hitze.

Das folgende Beispiel erläutert die Erfindung, schränkt sie jedoch nicht ein.

### Beispiel 1

### Herstellung einer mikrobiellen Antigenzusammensetzung zum Vorbeugen und Behandeln allergischer Erkrankungen

Luftpartikel aus einem Rinderstall wurden mit einem Impaktor (Andersen Instrument Incorporated's Six Stage Viable lmpactor), bei einer Durchflussrate von 28.3 Litern pro Minute Ober einen Zeitraum von 4 Stunden, 3 mal in der Woche über einen Monat hinweg gesammelt Nach jeder Messung wurde die mikrobielle Belastung der Stalltuft durch das Bebrüten der Agarplatten aus dem Impaktors bestimmt Die Anzahl und Spezies der koloniebildenden Keime aller in diesem Zeitraum genommener Proben wurde ermittelt und somit eine durchschnittliche Zusammensetzung der Stallluft bestimmt Diese ermittelte Zusammensetzung wurde aus Flüssigkulturen der Mikroorganismen rekonstruiert, indem die Kulturen ihren jeweilig gemessenen Anteilen gemäß zusammengegeben wurden. Die so erhaltene Suspension wurde abzentrifugiert und das Pellet mit den Mikroorganismen wurde mehrfach mit PBS gewaschen und durch Kältebehandlung sterilisiert.

## Patentansprüche

1. Verwendung einer Zusammensetzung, die aerogen übertragbare mikrobielle Antigene umfasst, wobei die mikrobiellen Antigene ein oder mehrere bakterielle Antigene aus *Bordetella spp, Brucella spp, Corynebacterium spp, Erysipelothrix spp, Escherichia spp, Haemophilus spp, Listeria spp, Leptospira spp, Mycobacterium spp, Mycoplasma spp, Pasteurella spp, Salmonella spp, Streptococcus spp* und *Coxiella spp* oder einer Kombination daraus umfassen, und weiterhin ein oder mehrere Antigene umfassen, die aus der Pilze, Viren und Protozoen und Fragmenten davon umfassende Gruppe ausgewählt sind, **dadurch gekennzeichnet, dass** sie hauptsächlich isolierte Fragmente enthält, zur Herstellung eines Arzneimittels zum Vorbeugen oder Behandeln von allergischen Erkrankungen.

2. Verwendung gemäß Anspruch 1, **dadurch gekennzeichnet, dass** die Fragmente allein oder in Kombination ausgewählt sind aus Zelltrümmern, Membranstücken, Organellen, Proteinen, Peptiden, Membranlipiden oder Endotoxinen.

3. Verwendung gemäß Anspruch 2, **dadurch gekennzeichnet, dass** die Proteine ausgewählt sind aus viralen Hüllproteinen, Zellwandproteinen, cytosolischen Proteinen und Glycoproteinen und/oder die Endotoxine ausgewählt sind aus Lipopolysaccharid, Staphylococcus Endotoxin, Lipoteichonsäure und Lipoarabinomannan.

4. Verwendung gemäß Anspruch 1 bis 3, **dadurch gekennzeichnet, dass** die Zusammensetzung als wässrige Lösung, Lypophylisat oder als Aerosol vorliegt.

5. Verwendung gemäß einem der Ansprüche 1 bis 4, **dadurch gekennzeichnet, dass** das Arzneimittel zum Vorbeugen oder Behandeln von Heuschnupfen, atopischer Dermatitis oder Asthma geeignet ist.

6. Verwendung gemäß einem der Ansprüche 1 bis 5, **dadurch gekennzeichnet, dass** das Arzneimittel zum Vorbeugen von allergischen Erkrankungen bei Säuglingen, die auf Grund einer positiven Familienanamnese ein deutlich höheres Risiko aufweisen, eine allergische Erkrankung zu entwickeln, geeignet ist.

7. Verwendung gemäß einem der Ansprüche 1 bis 6, **dadurch gekennzeichnet, dass** das Arzneimittel zum Vorbeugen oder Behandeln von allergischen Erkrankungen bei Säuglingen, die erste Manifestationen einer Allergie aufweisen, geeignet ist.

8. Verfahren zum Herstellen einer Zusammensetzung wie sie in einem der Ansprüche 1 bis 7 definiert ist, **dadurch gekennzeichnet, dass** Mikroorganismen aus Stallluft gesammelt und aufgeschlossen werden.

9. Verfahren gemäß Anspruch 8, **dadurch gekennzeichnet, dass** zum Sammeln der Mikroorganismen ein Impaktor, ein Impinger oder ein Luftfilter verwendet wird.

10. Verfahren gemäß Anspruch 9, **dadurch gekennzeichnet, dass** ein Kaskadenimpaktor oder ein Membranfilter verwendet wird.

11. Verfahren gemäß Anspruch 8 bis 10, **dadurch gekennzeichnet, dass** es zusätzlich ein Sterilisationsverfahren umfasst.

12. Verfahren gemäß Anspruch 8 bis 11, **dadurch gekennzeichnet, dass** die Zusammensetzung mit einem fungiziden oder fungistatischen Mittel behandelt wird.

13. Verfahren gemäß Anspruch 8 bis 12, **dadurch gekennzeichnet, dass** die Zusammensetzung mit einem viriziden oder virustatischen Mittel behandelt wird.

14. Verfahren gemäß Anspruch 8 bis 13, **dadurch gekennzeichnet, dass** die Zusammensetzung mit einem bakteriziden oder bakteriostatischen Mittel behandelt wird.

## Claims

1. Use of a composition comprising aerogenically transmissible microbial antigens, the latter comprising one or more bacterial antigens selected from *Bordetella spp, Brucella spp, Corynebacterium spp, Erysipelothrix spp, Escherichia spp, Haemophilus spp, Listeria spp, Leptospira spp, Mycobacterium spp, Mycoplasma spp, Pasteurella spp, Salmonella spp, Streptococcus spp* and *Coxiella spp,* or a combination thereof, together with one or more antigens selected from the group comprising fungi, viruses, protozoa and fragments thereof, **characterized in that** it contains mainly isolated fragments, for the preparation of a drug for the prevention or treatment of allergic diseases.

2. Use according to Claim 1, **characterized in that** the fragments are selected individually or in combination from cell detritus, pieces of membrane, organelles, proteins, peptides, membrane lipids and endotoxins.

3. Use according to Claim 2, **characterized in that** the proteins are selected from viral coat proteins, cell wall proteins, cytosol proteins and glycoproteins and/or the endotoxins are selected from lipopolysaccharide, Staphylococcus endotoxin, lipoteichonic acid and lipoarabinomannan.

4. Use according to Claims 1 to 3, **characterized in that** the composition is in the form of an aqueous solution, a lyophilizate or an aerosol.

5. Use according to one of Claims 1 to 4, **characterized in that** the drug is suitable for the prevention or treatment of hay fever, atopic dermatitis or asthma.

6. Use according to one of Claims 1 to 5, **characterized in that** the drug is suitable for the prevention of allergic diseases in newborns who, because of a positive family case history, have an appreciably higher risk of developing an allergic disease.

7. Use according to one of Claims 1 to 6, **characterized in that** the drug is suitable for the prevention or treatment of allergic diseases in newborns exhibiting primary manifestations of an allergy.

8. Process for the preparation of a composition as defined in one of Claims 1 to 7, **characterized in that** microorganisms are collected from cowshed air and digested.

9. Process according to Claim 8, **characterized in that** the microorganisms are collected using an impactor, an impinger or an air filter.

10. Process according to Claim 9, **characterized in that** a cascade impactor or a membrane filter is used.

11. Process according to Claims 8 to 10, **characterized in that** it additionally comprises a sterilization process.

12. Process according to Claims 8 to 11, **characterized in that** the composition is treated with a fungicidal or fungistatic agent.

13. Process according to Claims 8 to 12, **characterized in that** the composition is treated with a virucidal or virustatic agent.

14. Process according to Claims 8 to 13, **characterized in that** the composition is treated with a bactericidal or bacteriostatic agent.

## Revendications

1. Utilisation d'une composition, qui comprend des antigènes microbiens transmissibles par voie aérienne, lesdits antigènes microbiens comprenant un ou plusieurs antigènes bactériens de *Bordetella spp, Brucella spp, Corynebacterium spp, Erysipelothrix spp, Escherichia spp, Haemophilus spp, Listeria spp, Leptospira spp, Mycobacterium spp, Mycoplasma spp, Pasteurella spp, Salmonella spp, Streptococcus spp* et *Coxiella spp* ou une combinaison de ceux-ci, et comprenant de plus un ou plusieurs antigènes qui sont choisis dans le groupe des champignons, des virus et des protozoaires et des fragments de ceux-ci, **caractérisée en ce qu'**elle contient principalement des fragments isolés, pour la préparation d'un médicament destiné à la prévention ou au traitement de maladies allergiques.

2. Utilisation selon la revendication 1, **caractérisée en ce que** les fragments seuls ou en combinaison sont choisis parmi les déchets cellulaires, les fragments membranaires, les organelles, les protéines, les peptides, les lipides membranaires ou les endotoxines.

3. Utilisation selon la revendication 2, **caractérisée en ce que** les protéines sont choisies parmi les protéines d'enveloppe virales, les protéines de paroi cellulaire, les protéines cytosoliques, les glycoprotéines et/ou **en ce que** les endotoxines sont choisies parmi le lipopolysaccharide, l'endotoxine de staphylococcus, l'acide lipoteichoique, le lipoarabinomannane.

4. Utilisation selon l'une quelconque des revendications 1 à 3, **caractérisée en ce que** la composition se présente sous la forme d'une solution aqueuse, d'un lyophilisat ou d'un aérosol.

5. Utilisation selon l'une quelconque des revendications 1 à 4, **caractérisée en ce que** le médicament convient pour la prévention ou le traitement de la pollinose, de la dermatite atopique ou de l'asthme.

6. Utilisation selon l'une quelconque des revendications 1 à 5, **caractérisée en ce que** le médicament est adapté à la prévention de maladies allergiques chez les nourrissons qui en raison d'une anamnèse familiale positive présentent un risque sensiblement élevé de développer une maladie allergique.

7. Utilisation selon l'une quelconque des revendications 1 à 6, **caractérisée en ce que** le médicament est adapté à la prévention ou le traitement de maladies allergiques chez les nourrissons qui présentent les premières manifestations d'une allergie.

8. Procédé de fabrication d'une composition telle que définie dans l'une quelconque des revendications 1 à 7, **caractérisé en ce que** des microorganismes sont collectés de l'air ambiant du local de stabulation et sont lysés.

9. Procédé selon la revendication 8, **caractérisé en ce qu'**un impacteur, un appareil de mesure de Greenburg-Smith ou un filtre à air sont utilisés pour collecter les microorganismes.

10. Procédé selon la revendication 9, **caractérisé en ce qu'**un impacteur en cascade ou un filtre à membrane est utilisé.

11. Procédé selon l'une quelconque des revendications 8 à 10, **caractérisé en ce qu'**il comprend en plus un procédé de stérilisation.

12. Procédé selon l'une quelconque des revendications 8 à 11, **caractérisé en ce que** la composition est traitée avec un produit fongicide ou fongistatique.

13. Procédé selon l'une quelconque des revendications 8 à 12, **caractérisé en ce que** la composition est traitée avec un produit viricide ou virostatique.

14. Procédé selon l'une quelconque des revendications 8 à 13, **caractérisé en ce que** la composition est traitée avec un produit bactéricide ou bactériostatique.
